# EUROPÄISCHE PATENTSCHRIFT

(11) **EP 2 339 968 B1**
(45) Veröffentlichungstag und Bekanntmachung des Hinweises auf die Patenterteilung: **14.11.2012**
(21) Anmeldenummer: 10703413.4
(22) Anmeldetag: 01.02.2010
(51) Int. Cl.: A61B 8/12, A61B 8/14, G01S 15/89, A61B 5/06

(54) **SENSORSYSTEM UND VERFAHREN ZUR BILDGEBENDEN ERFASSUNG EINES OBJEKTES**
SENSOR SYSTEM AND METHOD FOR IMAGE CAPTURE OF AN OBJECT
SYSTEME CAPTEUR ET PROCEDE DE DETECTION D'UN OBJET AVEC FORMATION D'UNE IMAGE

(30) Priorität: 06.02.2009 DE 102009007868
(43) Veröffentlichungstag der Anmeldung: 06.07.2011
(73) Patentinhaber: Fraunhofer-Gesellschaft zur Förderung der angewandten Forschung e.V., 80686 München (DE); Friedrich-Alexander-Universität Erlangen-Nürnberg, 91054 Erlangen (DE)
(72) Erfinder: BERGEN, Tobias, 90425 Nürnberg (DE); MÜNZENMAYER, Christian, 90409 Nürnberg (DE); WINTER, Christian Dr.-Ing., 31141 Hildesheim (DE); WITTENBERG, Thomas, 91054 Erlangen (DE)
(74) Vertreter: Rau, Schneck & Hübner
(86) Internationale Anmeldenummer: PCT/EP2010/000574
(87) Internationale Veröffentlichungsnummer: WO 2010/089066

(56) Entgegenhaltungen:
- EP-A1- 1 504 721
- WO-A2-2006/127142
- DE-A1- 10 208 437
- US-A1- 2004 167 402
- US-A1- 2008 281 236
- US-B1- 6 554 771

## Beschreibung

Die Erfindung betrifft ein Sensorsystem zur bildgebenden Erfassung eines Objektes gemäß dem Oberbegriff des Anspruchs 1.

Bildgebende Verfahren zur Erfassung von Objekten haben in verschiedensten Anwendungsbereichen eine große Bedeutung gewonnen. Typische Anwendungsbereiche sind beispielsweise die Erfassung von Organen in der Medizin sowie die Material- und Bauteilprüfung in der Industrie. Beispielsweise können in der Medizin mittels eines Ultraschallsensors Gewebe in Form eines dreidimensionalen Bildes erfasst und so die Umrisse eines Tumors identifiziert werden, so dass ein gezielter Eingriff geplant und durchgeführt werden kann. Um die dreidimensionale Struktur des Objektes zu erfassen, müssen mehrere zweidimensionale Schnittbilder versetzt zueinander aufgenommen und anschließend zu einem dreidimensionalen Objektbild zusammengefügt werden. Die Qualität des dreidimensionalen Objektbildes hängt dabei von der Genauigkeit ab, mit der die einzelnen zweidimensionalen Schnittbilder zusammengefügt werden. Zum Zusammenfügen der einzelnen Schnittbilder ist es erforderlich, die Bewegung des Ultraschallsensors relativ zu dem Objekt zu bestimmen, da sie die Positionen und Orientierungen der einzelnen Schnittbilder zueinander wiedergibt.

Aus der EP 0 961 135 B1 ist ein Sensorsystem zur Aufnahme von Ultraschallschnittbildem bekannt, bei der an einer Ultraschallsonde ein Positionsfühler angeordnet ist, der es ermöglicht, die aufgenommenen Schnittbilder korrekt zueinander zu positionieren, wenn die Ultraschallsonde während der Aufnahme bewegt wird. Dadurch, dass der Positionsfühler absolute Positionsdaten in einem festen Bezugskoordinatensystem liefert, kann die Bewegung der Ultraschallsonde bei der Rekonstruktion eines dreidimensionalen Objektbildes aus den Schnittbildern berücksichtigt werden. Nachteilig ist, dass Eigenbewegungen des Objektes während der Aufnahme der Schnittbilder durch den Positionsfühler nicht erfasst werden können, da diese keinen Einfluss auf die Position der Ultraschallsonde haben. Dementsprechend werden durch Eigenbewegungen des Objektes verursachte Relativbewegungen zwischen der Ultraschallsonde und dem Objekt nicht erfasst und bei der Rekonstruktion des dreidimensionalen Objektbildes nicht berücksichtigt, was zu einer schlechten Bildqualität führt.

Aus der EP 1 504 721 A1 ist ein entsprechendes Sensorsystem bekannt, bei dem endseitig an der Ultraschallsonde zusätzlich ein optischer Sensor zur Aufnahme von Bilddaten des zu untersuchenden Objektes angeordnet ist.

Aus der US 2004/167 402 A1 ist ein Ultraschallgerät mit einer Ultraschallsonde und einem zugehörigen optischen Sensor bekannt. Der optische Sensor ist benachbart zu einem akustischen Fenster angeordnet, hinter dem die Ultraschallsonde liegt. Der optische Sensor ist derart ausgerichtet, dass dieser Licht von der Hautoberfläche eines Patienten empfängt. Aus den entsprechenden Bildern wird die Bewegung der Ultraschallsonde ermittelt. Die von dieser aufgenommenen Daten werden anhand der ermittelten Bilder zu einem Objektbild rekonstruiert.

Entsprechende Ultraschallgeräte sind aus der WO 2006/127 142 A2 und der US 6 554 771 B1 bekannt, die mittels eines optischen Sensors Positionsdaten einer Ultraschallsonde gewinnen und diese zur Auswertung der mittels der Ultraschallsonde aufgenommenen Daten verwenden. Nachteilig bei diesen Ultraschallgeräten ist, dass Eigenbewegungen des Objekts während der Aufnahme noch immer die Bildqualität beeinträchtigen.

Der Erfindung liegt daher die Aufgabe zugrunde, ein Sensorsystem zur bildgebenden Erfassung eines Objektes zu schaffen, mittels dem durch Eigenbewegungen des Objektes verursachte Relativbewegungen zwischen einem bildgebenden Sensor und dem Objekt in einfacher Weise besser erfassbar sind.

Diese Aufgabe wird durch ein Sensorsystem mit den Merkmalen des Anspruchs 1 gelöst. Durch den optischen Sensor können von dem bildgebenden Sensor und dem zu erfassenden Objekt Bilddaten aufgenommen werden. Dabei ist aufgrund der ortsfesten Anordnung des bildgebenden Sensors relativ zu dem optischen Sensor sichergestellt, dass diese zueinander im Wesentlichen keine Relativbewegung ausführen können. Parallel zu den Bilddaten können mittels des bildgebenden Sensors bildgebende Daten des Objektes aufgenommen werden, so dass jeweils zu gleichen Zeitpunkten bildgebende Daten und Bilddaten vorliegen. Mittels der Auswerteeinheit kann aus den Bilddaten zu aufeinanderfolgenden Zeitpunkten die Relativbewegung zwischen dem bildgebenden Sensor und dem Objekt berechnet werden. Hierbei werden sowohl Relativbewegungen berücksichtigt, die durch eine Eigenbewegung des bildgebenden Sensors verursacht werden, als auch Relativbewegungen, die durch Eigenbewegungen des Objektes verursacht werden. Die bildgebenden Daten zu den entsprechenden Zeitpunkten können einander anhand der berechneten Relativbewegungen räumlich exakt zugeordnet werden. Hierdurch ist die Rekonstruktion von dreidimensionalen Objektbildern auch bei Eigenbewegungen des Objektes mit einer hohen Genauigkeit möglich, so dass eine hohe Bildqualität erzielbar ist.

Bei den Eigenbewegungen kann es sich um eine Bewegung des gesamten Objektes handeln, wie beispielsweise die Bewegung von Organen aufgrund der Atmung oder Durchblutung, oder um eine Deformation des Objektes, wie beispielsweise der Deformation eines Organs aufgrund einer Fremdeinwirkung. Bei der Fremdeinwirkung kann es sich insbesondere auch um eine Einwirkung des Sensorsystems handeln, das während der Aufnahme der bildgebenden Daten mit dem Objekt in Kontakt kommt. Derartige Deformationen können anhand der Bilddaten erkannt werden, so dass die zugehörigen bildgebenden Daten, welche aufgrund der Deformation des Objektes in der Regel unbrauchbar sind, entweder bei der Rekonstruktion des dreidimensionalen Objektbildes verworfen werden können oder - sofern die Bilddaten in Echtzeit vorliegen - zugehörige bildgebende Daten überhaupt nicht aufgenommen werden, bevor eine geeignete Aufnahmeposition des bildgebenden Sensors ohne Deformation des Objektes gefunden wurde.

Der bildgebende Sensor kann zur Aufnahme von zweidimensionalen Schnittbildern oder von dreidimensionalen Volumensegmentbildern ausgebildet sein, die anhand der berechneten Relativbewegungen zu einem dreidimensionalen Objektbild zusammengesetzt werden können. Der bildgebende Sensor kann beispielsweise ein Ultraschallsensor sein.

Die Ermittlung der Relativbewegungen aus den Bilddaten kann pixelbasiert erfolgen, wobei zu den Pixeln örtliche Verschiebungen zu den zeitlich benachbarten Bilddaten berechnet werden. Alternativ kann die Ermittlung der Relativbewegungen merkmalsbasiert erfolgen, wobei in zu den Bilddaten zugehörigen Oberflächenbildern stabile Strukturen gesucht und deren örtliche Verschiebungen ermittelt werden.

Ein Sensorsystem nach Anspruch 2 gewährleistet aufgrund der starren mechanischen Verbindung eine ortsfeste Anordnung der Sensoren zueinander. Dies gilt insbesondere, wenn auf das Sensorsystem Kräfte einwirken.

Ein Sensorsystem nach Anspruch 3 gewährleistet eine hohe mechanische Starrheit, da das Verbindungselement nahe bei den Sensoren angeordnet ist.

Ein Sensorsystem nach Anspruch 4 eignet sich zur Erfassung von Organen in der Medizin.

Ein Sensorsystem nach Anspruch 5 ermöglicht die Aufnahme von zweidimensionalen Schnittbildern, die mittels der Auswerteeinheit zu einem dreidimensionalen Objektbild zusammengefügt werden können.

Ein Sensorsystem nach Anspruch 6 ermöglicht die Aufnahme von dreidimensionalen Volumensegmentbildern, die zu einem dreidimensionalen Objektbild zusammengefügt werden können, das ein größeres Gesamtvolumen des Objektes zeigt.

Ein Sensorsystem nach Anspruch 7 ermöglicht das Ermitteln von absoluten Positionsdaten des bildgebenden Sensors und somit einen Bezug des bildgebenden Sensors zu einem absoluten Koordinatensystem. Durch die Positionsmesseinrichtung können durch Eigenbewegungen des bildgebenden Sensors verursachte Relativbewegungen von durch Eigenbewegungen des Objektes verursachten Relativbewegungen unterschieden werden.

Ein Sensorsystem nach Anspruch 8 ist frei im Raum bewegbar. Im Gegensatz zu optisch ausgebildeten Positionsmesseinrichtungen ist zur Positionsmessung keine optische Sichtverbindung erforderlich.

Ein Sensorsystem nach Anspruch 9 ermöglicht eine Ermittlung von absoluten Positionsdaten des bildgebenden Sensors mit einer hohen Genauigkeit. Die elektromagnetische Sonde kann beispielsweise an dem bildgebenden Sensor direkt angeordnet sein. Alternativ kann die elektromagnetische Sonde an dem Verbindungselement oder dem optischen Sensor angeordnet sein, da diese starr und in bekannten Abständen mit dem bildgebenden Sensor verbunden sind.

Ein Sensorsystem nach Anspruch 10 ermöglicht eine Ermittlung der Relativbewegung mit einer hohen Genauigkeit, da die erfassten absoluten Positionsdaten mit der Relativbewegung ergänzt werden. Beispielsweise kann eine präzisere Ermittlung der Relativbewegung derart erfolgen, dass anhand der absoluten Positionsdaten zunächst eine grobe Positionsbestimmung des bildgebenden Sensors vorgenommen wird, die mit der ermittelten Relativbewegung kombiniert wird. Fehler bei der Ermittlung der Relativbewegung, die bei pixel- oder merkmalsbasierten Verfahren beispielsweise bei weitreichenden Bewegungen auftreten können, können mittels des Abgleichs mit den absoluten Positionsdaten erkannt und korrigiert werden. Bei bildgebenden Sensoren, die in der Regel direkt auf dem Objekt aufliegen oder in einem Abstand über das Objekt geführt werden, muss bei der Ermittlung der Relativbewegungen insbesondere die Translation und Rotation in einer tangential zu der Oberfläche des Objektes liegenden Ebene und eine Kippbewegung relativ zu dem Objekt erfasst werden. Hierbei ist ein Abgleich zwischen der ermittelten Relativbewegung und den absoluten Positionsdaten vorteilhaft.

Ein Endoskopsystem nach Anspruch 11 ermöglicht eine Erfassung von schwer zugänglichen Stellen des Objektes. Der für die Ermittlung der Relativbewegungen relevante optische Sensor ist bei dem Endoskopsystem unmittelbar am Punkt der tatsächlichen Bewegung, also dem zweiten Ende, angeordnet, wodurch die Relativbewegungen mit hoher Genauigkeit bestimmbar sind.

Ein Endoskopsystem nach Anspruch 12 ermöglicht eine verbesserte Zugänglichkeit von schwer zugänglichen Stellen des Objektes. Ungenauigkeiten bei der Ermittlung der Relativbewegungen durch Verbiegen des flexibel ausgebildeten Schaftes und/oder Vibrationen treten aufgrund der Anordnung des optischen Sensors an dem zweiten Ende nicht auf.

Weitere Merkmale, Vorteile und Einzelheiten der Erfindung ergeben sich aus der nachfolgenden Beschreibung eines Ausführungsbeispiels anhand der Zeichnung. Es zeigen:
- Fig. 1: eine perspektivische Darstellung eines Endoskopsystems mit einem einen bildgebenden Sensor und einen optischen Sensor umfassenden Sensorsystem,
- Fig. 2: eine schematische Darstellung von mittels des bildgebenden Sensors aufgenommenen Schnittbildern und mittels des optischen Sensors aufgenommenen Oberflächenbildern,
- Fig. 3: eine schematische Darstellung von zeitlich aufeinanderfolgenden und deckungsgleich angeordneten Oberflächenbildern zur Ermittlung von Relativbewegungen zwischen dem bildgebenden Sensor und dem Objekt,
- Fig. 4: eine perspektivische Darstellung des Endoskopsystems in Fig. 1 in einer das Objekt deformierenden Position, und

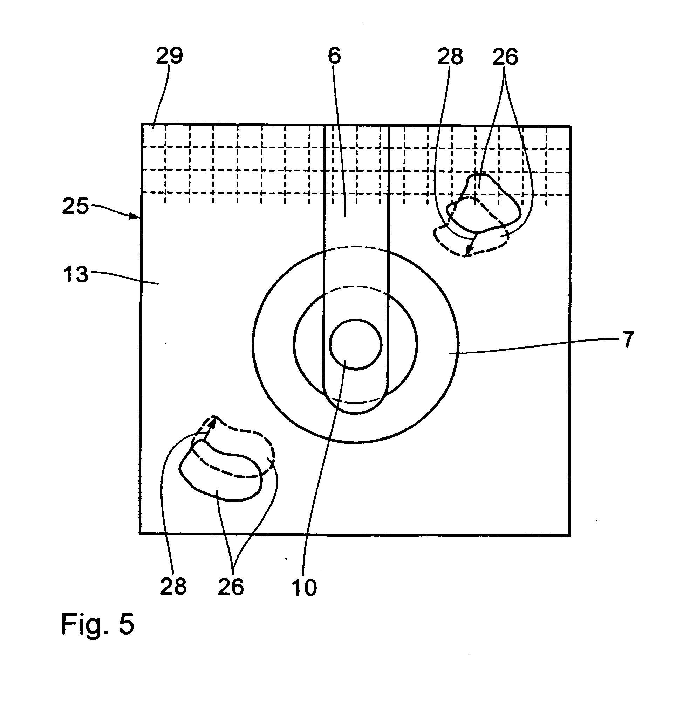

- Fig. 5: eine schematische Darstellung von zwei zeitlich aufeinanderfolgenden Oberflächenbildern entsprechend Fig. 3 bei einer Deformation des Objektes.

Ein Endoskopsystem 1 weist einen Schaft 2 auf, an dem an einem ersten Ende 3 ein Griff 4 angeordnet ist. Der Schaft 2 ist an einem zweiten Ende 5 V-förmig ausgebildet. An einem ersten Schaftabschnitt 6 ist ein bildgebender erster Sensor 7 angeordnet. Entsprechend ist an einem zweiten Schaftabschnitt 8 ein optischer zweiter Sensor 9 angeordnet. Zwischen den Schaftabschnitten 6, 8 verläuft nahe den Sensoren 7, 9 ein starres mechanisches Verbindungselement 10, das die Sensoren 7, 9 fest miteinander verbindet. Die Sensoren 7, 9 sind somit relativ zueinander im Wesentlichen ortsfest angeordnet. Die Schaftabschnitte 6, 8 sind flexibel ausgebildet, so dass die ortsfest miteinander verbundenen Sensoren 7, 9 an dem zweiten Ende 5 des Schaftes 2 beweglich angeordnet sind.

Der bildgebende Sensor 7 ist als Ultraschallsensor ausgebildet und Teil einer nicht näher dargestellten Ultraschallsonde. Der bildgebende Sensor 7 ist derart ausgebildet, dass bildgebende Daten für zweidimensionale Schnittbilder 11 und/oder dreidimensionale Volumensegmentbilder 12 eines zu erfassenden Objektes 13 aufnehmbar sind.

Der optische Sensor 9 ist als miniaturisierter Videosensor, beispielsweise in Tip-Chip-Technologie, ausgebildet. Der optische Sensor 9 weist einen dem bildgebenden Sensor 7 zugewandten Aufnahmebereich 14 auf, in dem der bildgebende Sensor 7 und teilweise das Verbindungselement 10 angeordnet sind. Dadurch, dass der bildgebende Sensor 7 in dem Aufnahmebereich 14 liegt, ist eine Aufnahme von Bilddaten des bildgebenden Sensors 7 und bei entsprechender Positionierung des bildgebenden Sensors 7 auch des Objektes 13 möglich. Der optische Sensor 9 stellt somit eine Kamera zur Beobachtung des bildgebenden Sensors 7.und des Objektes 13 dar.

Die Sensoren 7, 9 sind über in dem Schaft 2 geführte Signalleitungen 15 mit einer Auswerteeinheit 16 verbunden. Entsprechend sind die Sensoren 7, 9 über nicht näher dargestellte Versorgungsleitungen mit einer Energieversorgungseinheit verbunden.

Die Auswerteeinheit 16 dient zur Auswertung der aufgenommenen bildgebenden Daten sowie der aufgenommenen Bilddaten. Die Auswerteeinheit 16 ist derart ausgebildet, dass aus den Bilddaten Relativbewegungen zwischen dem bildgebenden Sensor 7 und dem Objekt 13 ermittelbar sind und in Abhängigkeit der ermittelten Relativbewegungen aus den bildgebenden Daten ein dreidimensionales Objektbild 17 des Objektes 13 rekonstruierbar ist.

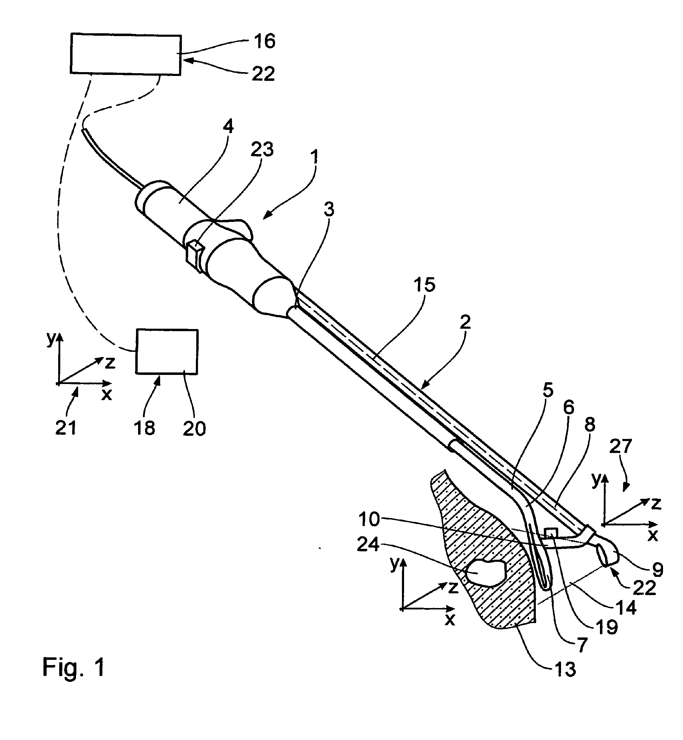

Zur Ermittlung von absoluten Positionsdaten des bildgebenden Sensors 7 ist eine Positionsmesseinrichtung 18 vorgesehen. Die Positionsmesseinrichtung 18 ist elektromagnetisch ausgebildet und weist eine als Spule ausgebildete elektromagnetische Sonde 19 und eine Sondendetektionseinheit 20 auf. Die Sonde 19 ist im Bereich der starr verbundenen Sensoren 7, 9 angeordnet. Figur 1 zeigt beispielsweise eine Anordnung der Sonde 19 an dem Verbindungselement 10. Die Sondendetektionseinheit 20 dient zur Erzeugung eines Magnetfeldes, in dem die Absolutposition der Sonde 19 in einem ersten kartesischen Koordinatensystem 21 ermittelbar ist. Die Sondendetektionseinheit 20 ist über Signalleitungen 22 mit der Auswerteeinheit 16 verbunden, so dass mittels der Auswerteeinheit 16 die Relativbewegungen zwischen dem bildgebenden Sensor 7 und dem Objekt 13 unter Berücksichtigung der absoluten Positionsdaten des bildgebenden Sensors 7 ermittelbar sind.

Der bildgebende Sensor 7, der optische Sensor 9, das Verbindungselement 10, die Auswerteeinheit 16 und die Positionsmesseinrichtung 18 bilden zusammen ein Sensorsystem 22. Das Sensorsystem 22 ist Teil des Endoskopsystems 1. Das Endoskopsystem 1 weist im Bereich des Griffes 4 mehrere Bedienelemente 23 zur Bedienung der Sensoren 7, 9 auf.

Nachfolgend ist die Funktionsweise des Endoskopsystems 1 und des Sensorsystems 22 beschrieben. Mittels des Endoskopsystems 1 werden die Sensoren 7, 9 in die unmittelbare Nähe des Objektes 13 gebracht. Das Objekt 13 kann beispielsweise ein zu erfassendes Organ sein, das eine Eigenbewegung ausführt. Eigenbewegungen können beispielsweise eine gesamte Bewegung des Organs aufgrund der Atmung oder eine Bewegung von Organteilbereichen aufgrund des Einwirkens von Kräften sein.

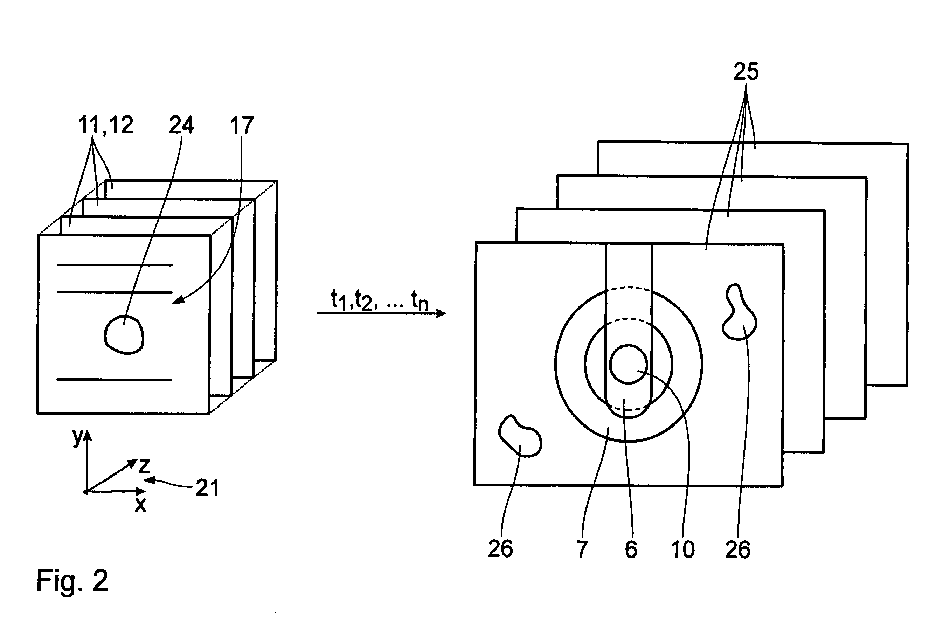

Figur 1 zeigt die Sensoren 7, 9 in einer Aufnahmeposition. In dieser Aufnahmeposition werden mittels des bildgebenden Sensors 7 und des optischen Sensors 9 eine Sequenz von bildgebenden Daten und eine entsprechende Sequenz von Bilddaten aufgenommen. Bei der Aufnahme wird der bildgebende Sensor 7 in dem ersten Koordinatensystem 21 räumlich verschoben, so dass in einer ersten Aufnahmeposition erste bildgebende Daten, in einer zweiten Aufnahmeposition zweite bildgebende Daten und in einer n-ten Aufnahmeposition zugehörige n-te bildgebende Daten aufgenommen werden, die an die Auswerteeinheit 16 übermittelt werden. Die bildgebenden Daten enthalten Informationen für ein entsprechendes erstes bis n-tes Schnittbild 11. Somit wird mittels des bildgebenden Sensors 7 eine Sequenz von Schnittbildern 11 in verschiedenen Aufnahmepositionen aufgenommen. Die einzelnen Schnittbilder 11 werden dabei zu unterschiedlichen Zeitpunkten t aufgenommen, wobei das erste Schnittbild 11 zu einem ersten Zeitpunkt t₁, das zweite Schnittbild 11 zu einem zweiten Zeitpunkt t₂ und das n-te Schnittbild 11 zu einem n-ten Zeitpunkt tₙ aufgenommen wird. Figur 2 zeigt beispielhaft die Sequenz von Schnittbildern 11, die bei einer räumlichen Verschiebung in einer z-Richtung aufgenommen wurden. In den Schnittbildern 11 ist eine unterhalb der Objektoberfläche liegende Struktur 24 erkennbar.

Die Schnittbilder 11 werden in der Auswerteeinheit 16 zu dem dreidimensionalen Objektbild 17 zusammengefügt. Hierzu müssen in der Auswerteeinheit 16 Informationen über die räumliche Lage der Schnittbilder 11 relativ zueinander vorliegen. Zur Gewinnung dieser Informationen werden zu den Zeitpunkten t₁ bis tₙ mittels des optischen Sensors 9 erste Bilddaten bis n-te Bilddaten aufgenommen. Die Bilddaten werden an die Auswerteeinheit 16 übertragen und in entsprechende Oberflächenbilder 25 der Objektoberfläche umgewandelt. Wie Figur 2 zeigt, sind auf den Oberflächenbildern 25 der bildgebende Sensor 7 mit dem Schaft 2 sowie Oberflächenstrukturen 26 abgebildet. Da der bildgebende Sensor 7 und der Schaft 2 in einem zweiten Koordinatensystem 27 des optischen Sensors 9 ortsfest angeordnet sind, charakterisieren Bewegungen der Oberflächenstrukturen 26 in der Sequenz von Oberflächenbildern 25 Relativbewegungen zwischen dem bildgebenden Sensor 7 und dem Objekt 13.

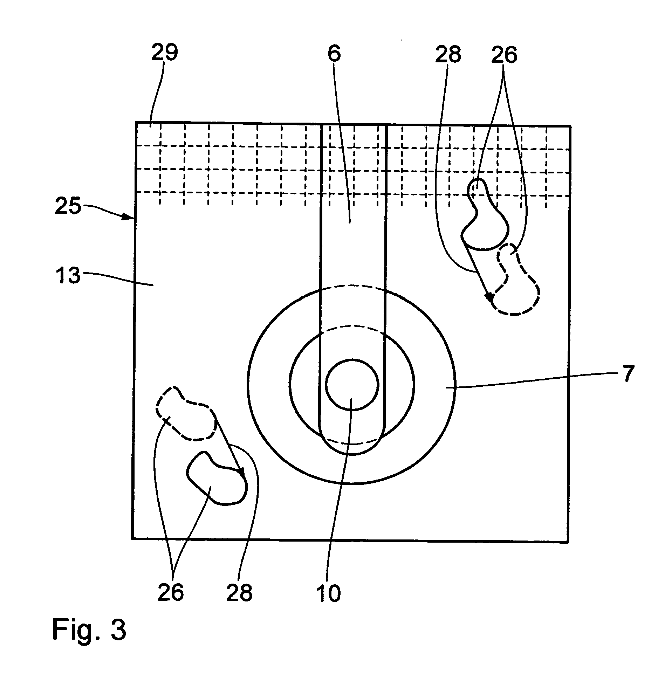

Zur räumlichen Zuordnung der Schnittbilder 11 werden aus den Oberflächenbildern 25 Relativbewegungen zwischen dem bildgebenden Sensor 7 und dem Objekt 13 ermittelt. Figur 3 zeigt zwei Oberflächenbilder 25 zu aufeinanderfolgenden Zeitpunkten t, die deckungsgleich übereinander gelegt sind. Die Oberflächenstrukturen 26 zu einem ersten Zeitpunkt, beispielsweise zu t₁, sind in Figur 3 mit einer durchgezogenen Linie dargestellt. Die Oberflächenstrukturen 26 zu einem nachfolgenden zweiten Zeitpunkt, beispielsweise zu t₂, sind in Figur 3 mit einer gestrichelten Linie dargestellt. Die Oberflächenstrukturen 26 weisen aufgrund von Bewegungen des bildgebenden Sensors 7 und/oder des Objektes 13 in dem zweiten Koordinatensystem 27, also in den Oberflächenbildern 25, unterschiedliche Positionen auf. Aus den unterschiedlichen Positionen der Oberflächenstrukturen 26 wird mittels der Auswerteeinheit 16 eine jeweilige Relativbewegung zwischen zwei aufeinanderfolgenden Zeitpunkten ermittelt.

Hierzu werden mittels eines pixelbasierten oder eines merkmalsbasierten Verfahrens ein oder mehrere Verschiebevektoren 28 bestimmt. Die Verschiebevektoren 28 charakterisieren die Relativbewegung zwischen dem bildgebenden Sensor 7 und dem Objekt 13. Auf Basis dieser Verschiebevektoren 28 werden die zugehörigen Schnittbilder 11 einander räumlich zugeordnet.

Bei dem pixelbasierten Verfahren erfolgt das Berechnen der Verschiebevektoren 28 auf der Basis von einzelnen Pixeln 29, die in Figur 3 angedeutet sind. Hinsichtlich der Einzelheiten von pixelbasierten Verfahren wird auf den Fachartikel "All About Direct Methods" von M. Irani und P. Anandan (http://citeseerx.ist.psu.edu/viewdoc/summary?doi=10.1.1.37.2273) verwiesen.

Bei dem merkmalsbasierten Verfahren werden aus geeigneten Bildbereichen stabile Strukturen, wie beispielsweise die Oberflächenstrukturen 26, selektiert und in zeitlich benachbarten Oberflächenbildern 25 gesucht. Die stabilen Strukturen bilden die Grundlage für das Ermitteln der Verschiebevektoren 28. Hinsichtlich der Einzelheiten von merkmalsbasierten Verfahren wird auf den Fachartikel "Feature Based Methods for Structure and Motion Estimation" von P. H. S. Torr und A. Zisserman (http://citeseerx.ist.psu.edu/viewdoc/summary?doi=10.1.1.37.1517) verwiesen.

Merkmalsbasierte Verfahren weisen gegenüber pixelbasierten Verfahren eine vergleichsweise hohe Geschwindigkeit bei der Berechnung der Verschiebevektoren 28 auf. Demgegenüber kann mit pixelbasierten Verfahren auch dann eine ausreichend genaue Berechnung von Verschiebevektoren 28 erfolgen, wenn ein merkmalsbasiertes Verfahren keine stabilen Strukturen auffindet. Beispielsweise können pixelbasierte Verfahren im Bereich von Farbverläufen zugehörige Verschiebevektoren 28 bestimmen.

Während der Aufnahme der bildgebenden Daten werden mittels der Positionsmesseinrichtung 18 zu dem ersten Zeitpunkt t₁ bis zu dem n-ten Zeitpunkt tₙ absolute Positionsdaten des bildgebenden Sensors 7 ermittelt. Die Positionseinrichtung 18 ist als übliches elektromagnetisches Trackingsystem ausgebildet, das ein Magnetfeld erzeugt, in dem die als Spule ausgebildete Sonde 19 bewegt und mittels der Detektionseinheit 20 detektiert und geortet werden kann. Die absoluten Positionsdaten enthalten somit Informationen über die Position und Orientierung der Sonde 19 in dem ersten Koordinatensystem 21. Da die Sonde 19 einen konstanten definierten Abstand zu dem bildgebenden Sensor 7 aufweist, können mittels der Auswerteeinheit 16 aus den absoluten Positionsdaten der Sonde 19 die absoluten Positionsdaten des bildgebenden Sensors 7 berechnet werden.

Die absoluten Positionsdaten des bildgebenden Sensors 7 können in der Auswerteeinheit 16 zum Abgleich und/oder zur Ergänzung bei der Ermittlung der Relativbewegungen eingesetzt werden. Beispielsweise zeigen pixel- oder merkmalsbasierte Verfahren Probleme beim Bestimmen von weitreichenden Relativbewegungen. Dementsprechend ist es bei weitreichenden Relativbewegungen vorteilhaft, zunächst durch die absoluten Positionsdaten einen Startwert für die Relativbewegung zu bekommen, der als Ausgangspunkt für das pixelbasierte oder merkmalsbasierte Verfahren dient. Die absoluten Positionsdaten liefern somit einen groben Wert für die Relativbewegung, der durch das pixel- oder merkmalsbasierte Verfahren präzisiert wird. Die Kombination der absoluten Positionsdaten mit den Ergebnissen des pixel- oder merkmalsbasierten Verfahrens ermöglicht somit eine hochgenaue Ermittlung der Relativbewegungen. Durch die elektromagnetische Positionsmesseinrichtung 18 ist die Freihandbewegung des Endoskops 1 bei der Erfassung des Objektes 13 nicht beeinträchtigt.

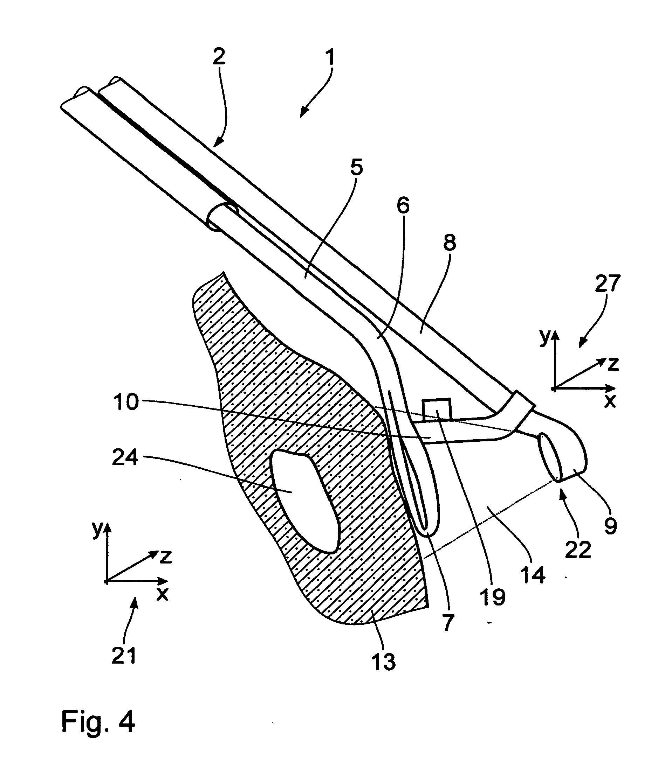

Beim Ermitteln der Relativbewegungen können auch durch Eigenbewegungen des Objektes 13 verursachte Relativbewegungen erfasst und ermittelt werden, so dass die Schnittbilder 11 örtlich exakt einander zugeordnet und zu dem dreidimensionalen Objektbild 17 zusammengefügt werden können. Darüber hinaus können in den Oberflächenbildern 25 Deformationen des Objektes 13 aufgrund von einwirkenden Kräften ermittelt werden. Figur 4 zeigt beispielsweise eine Deformation des Objektes 13, beispielsweise eines Organs, aufgrund des aufliegenden bildgebenden Sensors 7. Hierdurch wird auch die unter der Objektoberfläche liegende Struktur 24 deformiert, so dass in diesem Zustand aufgenommene Schnittbilder 11 nicht sinnvoll mit Schnittbildern 11 zusammengefügt werden können, die in einem nicht deformierten Zustand des Objektes 13 aufgenommen wurden. Dementsprechend müssen in dem deformierten Zustand aufgenommene Schnittbilder 11 mittels der Auswerteeinheit 16 entweder aussortiert werden oder überhaupt nicht aufgenommen werden. Deformationen können in den Oberflächenbildern 25 dadurch erkannt werden, dass die Oberflächenstrukturen 26 sich aufgrund der Deformation auf den bildgebenden Sensor 7 zubewegen. Bei der Ermittlung der Verschiebevektoren 28 ist dies dadurch erkennbar, dass diese auf den bildgebenden Sensor 7 zugerichtet sind. Erfolgt in der Auswerteeinheit 16 eine ständige Ermittlung der Relativbewegungen in Echtzeit, so kann aus der Richtung der Verschiebevektoren 28 eine Deformation erkannt und die Aufnahme eines entsprechenden Schnittbildes 11 vermieden werden. Anderenfalls kann das im deformierten Zustand aufgenommene Schnittbild 11 einfach erkannt und aussortiert werden.

Die obigen Ausführungen gelten entsprechend, wenn die bildgebenden Daten anstelle von Schnittbildern 11 dreidimensionale Volumensegmentbilder 12 charakterisieren, die zu dem größeren Objektbild 17 zusammengefügt werden.

Die Erfindung stellt ein bildgebendes Sensorsystem und ein entsprechendes bildgebendes Verfahren bereit, bei denen durch Eigenbewegungen des zu erfassenden Objektes 13 verursachte Relativbewegungen zwischen dem bildgebenden Sensor 7 und dem Objekt 13 ermittelt werden können. Mittels dieser Relativbewegungen können aufgenommene Schnittbilder 11 bzw. aufgenommene Volumensegmentbilder 12 einander räumlich zugeordnet und zu einem dreidimensionalen Objektbild 17 zusammengefügt werden. Da auch Eigenbewegungen des Objektes 13 berücksichtigt werden, ist die Zuordnung mit einer hohen Genauigkeit möglich, so dass eine entsprechend hohe Bildqualität des Objektbildes erzielt wird. Hierdurch können insbesondere dreidimensionale Bilder von dynamischen Organstrukturen mit einer hohen Bildqualität erstellt werden.

Aus den aufgenommenen Oberflächenbildern 25 zu aufeinanderfolgenden Zeitpunkten t werden jeweils Verschiebevektoren bzw. ein Verschiebevektorfeld ermittelt, das die räumliche Zuordnung der entsprechenden Schnittbilder 11 bzw. Volumensegmentbilder 12 beschreibt. Die Oberflächenbilder 25 werden mittels des optischen Sensors 9, der als miniaturisierter Videosensor ausgebildet ist, aufgenommen, so dass das Endoskopsystem 1 gleichzeitig ein Videoskop bereitstellt.

Durch die starre Verbindung der Sensoren 7, 9 ergibt sich eine Unabhängigkeit von externen Trackingmethoden, wie beispielsweise einem mechanischen oder optischen Tracking. Durch die lokale Berücksichtigung von Objektveränderungen, wie beispielsweise von Deformationen, können auch konventionelle Trackingmethoden ergänzt und/oder korrigiert werden. Die Erfindung weist eine hohe Toleranz gegenüber Eigenbewegungen des Objektes, wie beispielsweise Organbewegungen aufgrund von Kreislaufaktivitäten oder Atmung, auf.

Die Erfindung ist sowohl in der Medizin als auch in der Industrie einsetzbar. In der Medizin können - wie bereits ausgeführt - Organbewegungen berücksichtigt werden, wohingegen in der Industrie Bewegungen und/oder Verformungen von zu untersuchenden Bauteilen berücksichtigt werden können. In der Medizin kann das Endoskopsystem 1 bzw. das Sensorsystem 22 sowohl extrakorporal als auch intrakorporal angewandt werden. Beispielsweise kann eine Ultraschallsonde im B-Modus intrakorporal im Bauchraum zur Abbildung der Leber eingesetzt werden. Technische Anwendungen in der Industrie sind beispielsweise in der Werkstoff- und Materialprüfung, der Prüfung von technischen Textilien, Schäumen und Isoliermaterialien sowie von Bauteilen in der Luft- und Raumfahrttechnik und Rotorblättern von Windenergieanlagen. Die Prüfung kann insbesondere im laufenden Betrieb (Structural Health Monitoring) sowie an schwer zugänglichen Stellen erfolgen, wie beispielsweise in technischen Hohlräumen.

## Patentansprüche

1. Sensorsystem zur bildgebenden Erfassung eines Objektes mit
- einem bildgebenden Sensor (7) zur Aufnahme von bildgebenden Daten eines zu erfassenden Objektes (13),
- einem optischen Sensor (9) zur Aufnahme von Bilddaten des Objektes (13), wobei der bildgebende Sensor (7) relativ zu dem optischen Sensor (9) im Wesentlichen ortsfest angeordnet ist, und
- einer Auswerteeinheit (16) zur Auswertung der bildgebenden Daten und der Bilddaten, wobei die Auswerteeinheit (16) derart ausgebildet ist, dass
-- aus den Bilddaten eine Relativbewegung zwischen dem bildgebenden Sensor (7) und dem Objekt (13) ermittelbar ist, und
-- in Abhängigkeit der ermittelten Relativbewegung aus den bildgebenden Daten ein dreidimensionales Objektbild (17) rekonstruierbar ist,
**dadurch gekennzeichnet, dass**
- zur Aufnahme von Bilddaten des bildgebenden Sensors (7) der bildgebende Sensor (7) in einem für das Objekt (13) bestimmten Aufnahmebereich (14) des optischen Sensors (9) angeordnet ist und
- die Auswerteeinheit (16) derart ausgebildet ist, dass eine Deformation des Objektes (13) aufgrund einer Einwirkung des bildgebenden Sensors (7) anhand der Bilddaten erkennbar ist.

2. Sensorsystem nach Anspruch 1, **dadurch gekennzeichnet, dass** der bildgebende Sensor (7) mit dem optischen Sensor (9) durch ein starres Verbindungselement (10) fest verbunden ist.

3. Sensorsystem nach Anspruch 2, **dadurch gekennzeichnet, dass** das Verbindungselement (10) zumindest teilweise in dem Aufnahmebereich (14) angeordnet ist.

4. Sensorsystem nach einem der Ansprüche 1 bis 3, **dadurch gekennzeichnet, dass** der bildgebende Sensor (7) als Ultraschallsensor ausgebildet ist.

5. Sensorsystem nach einem der Ansprüche 1 bis 4, **dadurch gekennzeichnet, dass** der bildgebende Sensor (7) derart ausgebildet ist, dass bildgebende Daten für zweidimensionale Schnittbilder (11) des Objektes (13) aufnehmbar sind.

6. Sensorsystem nach einem der Ansprüche 1 bis 5, **dadurch gekennzeichnet, dass** der bildgebende Sensor (7) derart ausgebildet ist, dass bildgebende Daten für dreidimensionale Volumensegmentbilder (12) des Objektes (13) aufnehmbar sind.

7. Sensorsystem nach einem der Ansprüche 1 bis 6, **dadurch gekennzeichnet, dass** eine Positionsmesseinrichtung (18) zur Ermittlung von absoluten Positionsdaten des bildgebenden Sensors (7) vorgesehen ist.

8. Sensorsystem nach Anspruch 7, **dadurch gekennzeichnet, dass** die Positionsmesseinrichtung (18) elektromagnetisch ausgebildet ist.

9. Sensorsystem nach Anspruch 8, **dadurch gekennzeichnet, dass** die Positionsmesseinrichtung (18) eine elektromagnetische Sonde (19) aufweist, die im Bereich der starr verbundenen Sensoren (7, 9) angeordnet ist.

10. Sensorsystem nach einem der Ansprüche 7 bis 9, **dadurch gekennzeichnet, dass** die Auswerteeinheit (16) derart ausgebildet ist, dass die Relativbewegung zwischen dem bildgebenden Sensor (7) und dem Objekt (13) unter Berücksichtigung der absoluten Positionsdaten ermittelbar ist.

11. Endoskopsystem mit
- einem Schaft (2), welcher
-- ein einem zu erfassenden Objekt (13) abgewandtes erstes Ende (3) und
-- ein dem Objekt (13) zugewandtes zweites Ende (5) aufweist, und
- einem Sensorsystem (22) nach einem der Ansprüche 1 bis 10, wobei der bildgebende Sensor (7) und der optische Sensor (9) an dem zweiten Ende (5) angeordnet sind.

12. Endoskopsystem nach Anspruch 11, **dadurch gekennzeichnet, dass** der Schaft (2) zumindest im Bereich des zweiten Endes (5) flexibel ausgebildet ist.

## Claims

1. Sensor system for image capture of an object, the sensor system comprising
- an imaging sensor (7) for recording imaging data of an object (13) to be captured;
- an optical sensor (9) for recording image data of the object (13), with the imaging sensor (7) being arranged relative to the optical sensor (9) in such a way as to be essentially stationary; and
- an evaluation unit (16) for evaluating the imaging data and the image data, with the evaluation unit (16) being designed in such a way that
-- a relative movement between the imaging sensor (7) and the object (13) is determinable from the image data; and
-- a three-dimensional object image (17) is reconstructible from the imaging data as a function of the determined relative movement,
**characterized in that**
- for recording image data of the imaging sensor, the imaging sensor (7) is arranged in a recording area (14) of the optical sensor (9) which recording area (14) has been defined for the object (13) and
- the evaluation unit (16) is designed such that a deformation of the object (13) due to an influence of the imaging sensor (7) is recognizable by means of the image data.

2. Sensor system according to claim 1, **characterized in that** the imaging sensor (7) is securely connected to the optical sensor (9) by a rigid connection element (10).

3. Sensor system according to claim 2, **characterized in that** the connection element (10) is at least partially arranged in the recording area (14).

4. Sensor system according to one of claims 1 to 3, **characterized in that** the imaging sensor (7) is an ultrasonic sensor.

5. Sensor system according to one of claims 1 to 4, **characterized in that** the imaging sensor (7) is designed in such a way that imaging data for two-dimensional sectional images (11) of the object (13) are recordable.

6. Sensor system according to one of claims 1 to 5, **characterized in that** the imaging sensor (7) is designed in such a way that imaging data for three-dimensional volume segment images (12) of the object (13) are recordable.

7. Sensor system according to one of claims 1 to 6, **characterized in that** a position measuring device (18) is provided for determining absolute positional data of the imaging sensor (7).

8. Sensor system according to claim 7, **characterized in that** the position measuring device (18) is electromagnetic.

9. Sensor system according to claim 8, **characterized in that** the position measuring device (18) comprises an electromagnetic probe (19) which is arranged in the region of the rigidly connected sensors (7, 9).

10. Sensor system according to one of claims 7 to 9, **characterized in that** the evaluation unit (16) is designed in such a way that the relative movement between the imaging sensor (7) and the object (13) is determinable taking into account the absolute positional data.

11. Endoscopic system comprising
- a stem (2) which comprises
-- a first end (3) which faces away from an object (13) to be captured; and
-- a second end (5) which faces the object (13); and
- a sensor system (22) according to one of claims 1 to 10, with the imaging sensor (7) and the image sensor (9) being arranged at the second end (5).

12. Endoscopic system according to claim 11, **characterized in that** the stem (2) is flexible at least in the region of the second end (5).

## Revendications

1. Système capteur pour la détection d'un objet avec formation d'une image comprenant
- un capteur donnant une image (7) pour l'enregistrement de données avec formation d'images de l'objet à détecter (13),
- un capteur optique (9) pour l'enregistrement de données images de l'objet (13), le capteur donnant une image (7) étant disposé principalement à un lieu fixe par rapport au capteur optique (9), et
- une unité d'évaluation (16) pour l'exploitation des données donnant l'image et des données images, l'unité d'évaluation (16) étant conçue de telle manière
-- qu'un déplacement relatif entre le capteur donnant l'image (7) et l'objet (13) peut être détecté à partir des données images, et
-- qu'une image de l'objet en trois dimensions (17) peut être reconstruite à partir des données donnant l'image en fonction du déplacement relatif détecté,
**caractérisé en ce que**
- pour l'enregistrement de données images du capteur donnant l'image (7), le capteur donnant l'image (7) est disposé dans une zone d'enregistrement donnée du capteur optique (9) pour l'objet (13)
et
- l'unité d'évaluation (16) est conçue de telle manière qu'une déformation de l'objet (13) en raison d'un effet du capteur donnant l'image (7) peut être reconnue.

2. Système capteur selon la revendication 1 **caractérisé en ce que** le capteur donnant l'image (7) est relié solidement avec le capteur optique (9) au moyen d'un élément de liaison (10) rigide.

3. Système capteur selon la revendication 2 **caractérisé en ce que** l'élément de liaison (10) est disposé au moins partiellement dans la zone d'enregistrement (14).

4. Système capteur selon l'une des revendications de 1 à 3 **caractérisé en ce que** le capteur donnant l'image (7) est conçu sous la forme d'un capteur à ultrasons.

5. Système de capteur selon l'une des revendications de 1 à 4 **caractérisé en ce que** le capteur donnant l'image (7) est conçu de telle manière que des données donnant l'image peuvent être enregistrées pour des images de l'objet (13) en coupes bidimensionnelles (11).

6. Système de capteur selon l'une des revendications de 1 à 5 **caractérisé en ce que** le capteur donnant l'image (7) est conçu de telle manière que des données donnant l'image peuvent être enregistrées pour des images de segments volumiques tridimensionnels (12) de l'objet (13).

7. Système capteur selon l'une des revendications de 1 à 6 **caractérisé en ce qu'**un dispositif de mesure de la position (18) est prévu pour l'évaluation de données de position absolues du capteur donnant l'image (7).

8. Système capteur selon la revendication 7 **caractérisé en ce que** le dispositif de mesure de la position (18) est conçu sous une forme électromagnétique.

9. Système capteur selon la revendication 8 **caractérisé en ce que** le dispositif de mesure de position (18) présente une sonde (19) électromagnétique qui est disposée dans la zone des capteurs reliés de manière rigide (7, 9).

10. Système capteur selon l'une des revendications de 7 à 9 **caractérisé en ce que** l'unité d'évaluation (16) est conçue de telle manière que le mouvement relatif entre le capteur donnant l'image (7) et l'objet (13) peut être évalué en tenant compte des données de position absolues.

11. Système endoscopique comprenant
- une tige (2) qui
-- présente une première extrémité (3) orientée dans le sens opposé à un objet (13) à évaluer et
-- une seconde extrémité (5) orientée vers l'objet (13) et
- un système capteur (22) selon l'une des revendications de 1 à 10, le capteur donnant l'image (7) et le capteur optique (9) étant disposés à la seconde extrémité (5).

12. Système endoscopique selon la revendication 11 **caractérisé en ce que** la tige (2) est conçu de manière flexible au moins dans la zone de la seconde extrémité (5).
